# EUROPEAN PATENT APPLICATION

(11) **EP 4 682 901 A1**
(43) Date of publication of application: **21.01.2026**
(21) Application number: 25188093.6
(22) Date of filing: 08.07.2025
(51) Int. Cl.: G16H 20/70, A61B 5/00

(54) **METHOD AND DEVICE FOR ALLEVIATING COGNITIVE IMPAIRMENT BASED ON USER TERMINAL**

(30) Priority: 18.07.2024 KR 20240095185; 27.09.2024 KR 20240131971
(71) Applicant: Emocog Inc., Seoul 08708 (KR)
(72) Inventor: NOH, Yoo Hun, 11479 Gyeonggi-do (KR)
(74) Representative: Zacco Sweden AB

(57) **Abstract**

Provided is a method of alleviating cognitive impairment based on a user terminal, the method including performing control, in response to a first input to a user terminal, such that a first domain training algorithm, a second domain training algorithm, and a third domain training algorithm of a first phase are output on the user terminal, determining, based on second inputs to the user terminal, whether the first domain training algorithm, the second domain training algorithm, and the third domain training algorithm of the first phase are completed, and determining, based on a result of the determining, whether the first phase is normally terminated, performing control, after the first phase is terminated and in response to a third input to the user terminal, such that a first domain training algorithm, a second domain training algorithm, and a third domain training algorithm of a second phase are output on the user terminal, determining, based on fourth inputs to the user terminal, whether the first domain training algorithm, the second domain training algorithm, and the third domain training algorithm of the second phase are completed, and determining, based on a result of the determining, whether the second phase is normally terminated, and performing control, based on whether the first phase and the second phase are terminated, such that a first visual indication is output on the user terminal.

## Description

### BACKGROUND

### 1. Field

The present disclosure relates to a method and system for alleviating a patient's symptoms, and more particularly, to a method of configuring a digital dementia therapeutic capable of alleviating symptoms of a patient with cognitive impairment, based on a user terminal, and a device for implementing the method.

### 2. Description of the Related Art

The Republic of Korea is considered to have already entered an aging society. As the aging society progresses, the number of patients with dementia rapidly increases, and the social cost incurred to treat and care for patients with dementia inevitably increases gradually.

Training for stimulating cognitive function has already been proven effective in preventing dementia and alleviating the symptoms of already-developed dementia. In the case of patients with mild cognitive impairment, which corresponds to the pre-dementia stage, it has been proven through various papers that if patients with mild cognitive impairment receive training that may improve cognitive function, their cognitive function improves to a greater extent compared to patients with dementia.

### SUMMARY

A technical objective of the present disclosure is to provide a digital dementia therapeutic that may alleviate symptoms of a patient with cognitive impairment.

According to an embodiment of the present disclosure, a method includes performing control, in response to a first input to a user terminal, such that a first domain training algorithm, a second domain training algorithm, and a third domain training algorithm of a first phase are output on the user terminal, determining, based on second inputs to the user terminal, whether the first domain training algorithm, the second domain training algorithm, and the third domain training algorithm of the first phase are completed, and determining, based on a result of the determining, whether the first phase is normally terminated, performing control, after the first phase is terminated and in response to a third input to the user terminal, such that a first domain training algorithm, a second domain training algorithm, and a third domain training algorithm of a second phase are output on the user terminal, determining, based on fourth inputs to the user terminal, whether the first domain training algorithm, the second domain training algorithm, and the third domain training algorithm of the second phase are completed, and determining, based on a result of the determining, whether the second phase is normally terminated, and performing control, based on whether the first phase and the second phase are terminated, such that a first visual indication is output on the user terminal.

In the method, the first domain training algorithm, the second domain training algorithm, and the third domain training algorithm may each include two or more detailed training algorithms.

In the method, the first domain training algorithm may be an algorithm set to be arranged first in each of the first phase and the second phase.

In the method, the second domain training algorithm may be an algorithm set to be arranged and output immediately after the first domain training algorithm.

In the method, the second domain training algorithm may be an algorithm set to be arranged and output immediately before the third domain training algorithm.

In the method, the third domain training algorithm may be an algorithm set to be arranged and output immediately after the second domain training algorithm.

In the method, the first input for executing the first phase and the third input for executing the second phase may be inputs entered into the user terminal on a same date.

In the method, the first domain training algorithm of the second phase may be output two or more times within the second phase.

In the method, the first domain training algorithm of the second phase may be set to include two or more different detailed training algorithms to be consecutively output on the user terminal.

In the method, each time a training algorithm included in the first phase and the second phase is terminated, an assessment of a training result of a user for the training algorithm may be displayed through the user terminal in the form of a report.

In the method, each time a training algorithm included in the first phase and the second phase is terminated, assessments of training results of other users for the training algorithm, in addition to the assessment of the training result of the user, may be displayed through the user terminal in the form of a report.

In the method, an arrangement order of training algorithms included in the first phase and the second phase may be preset to maximize a training effect.

In the method, the performing of the control such that the first visual indication is output may include, based on the first phase and the second phase having been normally terminated on a same date consecutively for one week, integrating and outputting seven instances of the first visual indication, which are output for respective seven dates of the one week.

In the method, the first visual indication may be changed in response to an assessment of a training result in the first phase and the second phase.

In the method, the first phase may be performed in a morning, and the second phase may be performed in an afternoon.

In the method, the first domain training algorithm may include a detailed training algorithm for quantifying and assessing attentional control of a user, or for improving the attentional control of the user.

In the method, the second domain training algorithm may include a detailed training algorithm for quantifying and assessing associative skills of a user, or for improving the associative skills of the user.

In the method, the third domain training algorithm may include a detailed training algorithm for quantifying and assessing integrative skills of a user, or for improving the integrative skills of the user.

According to another embodiment of the present disclosure, a device includes a memory storing at least one program, and a processor configured to execute the at least one program to perform an operation, wherein the processor is further configured to perform control, in response to a first input to a user terminal, such that a first domain training algorithm, a second domain training algorithm, and a third domain training algorithm of a first phase are output on the user terminal, determine, based on second inputs to the user terminal, whether the first domain training algorithm, the second domain training algorithm, and the third domain training algorithm of the first phase are completed, determine, based on a result of the determining, whether the first phase is normally terminated, perform control, after the first phase is terminated and in response to a third input to the user terminal, such that a first domain training algorithm, a second domain training algorithm, and a third domain training algorithm of a second phase are output on the user terminal, determine, based on fourth inputs to the user terminal, whether the first domain training algorithm, the second domain training algorithm, and the third domain training algorithm of the second phase are completed, determine, based on a result of the determining, whether the second phase is normally terminated, and perform control, based on whether the first phase and the second phase are terminated, such that a first visual indication is output on the user terminal.

According to an embodiment of the present disclosure, provided is a computer-readable recording medium having stored therein a program for executing the method.

### BRIEF DESCRIPTION OF THE DRAWINGS

These and/or other aspects will become apparent and more readily appreciated from the following description of the embodiments, taken in conjunction with the accompanying drawings in which:
FIG. 1 is a diagram schematically illustrating an entire system for implementing the present disclosure;
FIG. 2 is a block diagram of a management server for implementing a method according to an embodiment of the present disclosure;
FIG. 3 is a diagram schematically illustrating a user terminal and a training program installed in the user terminal, according to an embodiment of the present disclosure;
FIG. 4 is a diagram for describing a first visual indication that is output through a user terminal;
FIG. 5 is a diagram schematically illustrating an example of a badge that is obtainable by a user;
FIG. 6 is a diagram schematically illustrating another example of a badge that is obtainable by a user;
FIG. 7 is a diagram schematically illustrating another example of a badge that is obtainable by a user;
FIG. 8 is a diagram for describing a bud of a flower of memory, which is an example of a first visual indication;
FIG. 9 is a diagram for describing a flower of memory in full bloom, which is another example of a first visual indication;
FIG. 10 is a diagram for describing a golden flower of memory in full bloom, which is another example of a first visual indication;
FIG. 11 is a diagram for describing archiving of a flower of memory;
FIG. 12 is a diagram for describing another embodiment of archiving of a flower of memory illustrated in FIG. 11; and
FIG. 13 is a flowchart of an example of a method according to the present disclosure.

### DETAILED DESCRIPTION

As the present disclosure allows for various changes and numerous embodiments, particular embodiments will be illustrated in the drawings and described in detail. The effects and features of the present disclosure and methods of achieving them will become clear with reference to the embodiments described in detail below with the drawings. However, the present disclosure is not limited to the embodiments disclosed below, and may be implemented in various forms.

Hereinafter, embodiments of the present disclosure will be described in detail with reference to the accompanying drawings, and the same or corresponding components will be denoted by the same reference numerals when described with reference to the accompanying drawings, and thus, their descriptions that are already provided will be omitted.

In the following embodiments, terms such as "first," "second," etc., are used only to distinguish one component from another, and such components must not be limited by these terms.

In the following embodiments, the singular expression also includes the plural meaning as long as it is not inconsistent with the context.

In the following embodiments, the terms "comprise," "include," "have," and the like specify the presence of stated features or components, but do not preclude the presence or addition of one or more other features or components.

When a certain embodiment may be differently implemented, particular operations may be performed differently from the sequence described herein. For example, two processes, which are successively described herein, may be substantially simultaneously performed, or may be performed in a process sequence opposite to a described process sequence.

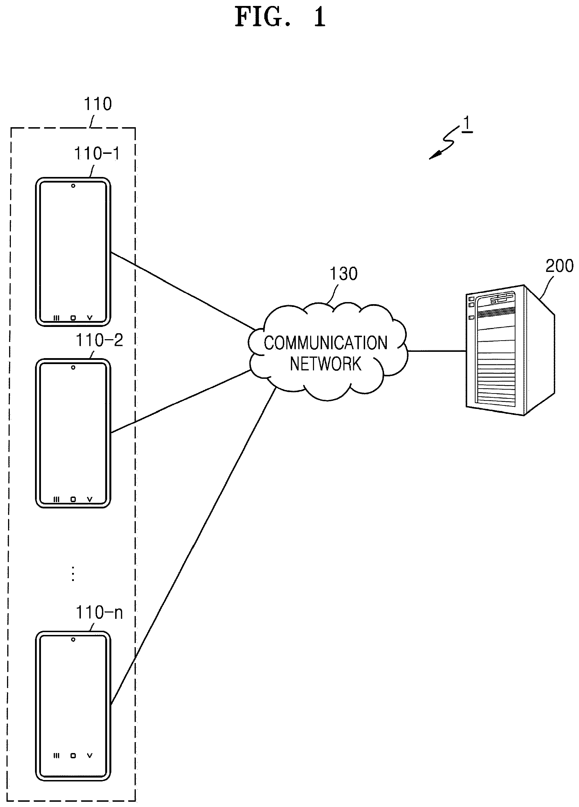

FIG. 1 is a diagram schematically illustrating an entire system for implementing the present disclosure.

Referring to FIG. 1, an entire system 1 according to the present disclosure has a structure in which a user terminal group 110 and a management server 200 are connected to each other through a communication network 130.

The user terminal group 110 may include one or more user terminals. For example, the number of user terminals included in the user terminal group 110 may be 1 or n as illustrated in FIG. 1. In FIG. 1, n may be an integer greater than or equal to 1.

Each user terminal included in the user terminal group 110 is a terminal of a user who uses a training program according to the present disclosure, and refers to an electronic device equipped with a communication module capable of communicating with the management server 200.

The user terminal refers to a smart device including an input device for receiving an input from the user, an output device (e.g., a display) for visually outputting an input to the user terminal or a result of processing by the user terminal, and a communication module capable of communicating with an external device, and thus, the size or type of the user terminal is not limited as long as it includes the input device, the output device, and the communication module. For example, although FIG. 1 illustrates that the user terminal is a smart phone, the user terminal may be a personal computer (PC), a notebook computer, a netbook, or the like capable of communicating with the management server 200.

The user of the user terminal refers to a person who uses a training program for alleviating the symptoms of a patient with cognitive impairment, and may be a patient diagnosed with cognitive impairment or a guardian of the patient. As another example, the user may be a tester for improving the performance of a training program by repeatedly executing the training program installed in the form of an application on the user terminal.

The management server 200 is a server in which an integrated management program is installed, and refers to a server configured to manage and control a data flow while communicating with a plurality of user terminals included in the user terminal group 110. The integrated management program (e.g., an integrated management app) is installed in the management server 200, and according to an embodiment, a part of the integrated management program may be implemented in the form of a client to be executed by a user terminal, and installed in the user terminals included in the user terminal group 110.

The communication network 130 performs a function of connecting the user terminals included in the user terminal group 110 to the management server 200, and may include various wired and wireless communication networks such as a data network, a mobile communication network, or an Internet network.

In the present disclosure, a training program refers to a logical device for controlling a screen output on the user terminal included in the user terminal group 110, under control of the management server 200. The training program does not have a physical form and may be optimized for a user of the training program multiple times. That is, the training program may be continuously updated based on an input by the user through the user terminal and a command received from the management server 200. Patients experiencing cognitive impairment may alleviate their cognitive impairment symptoms to a certain extent by repeatedly using a training program running on a user terminal.

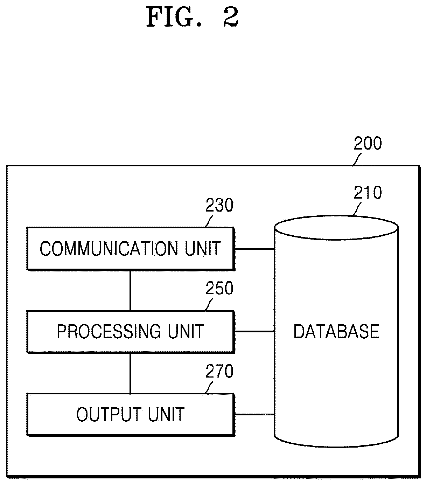

In the present disclosure, a training program may be composed of a first phase and a second phase. When the user executes the training program through the user terminal, training algorithms according to the first phase are initially output, followed by training algorithms according to the second phase, such that the user's cognitive impairment symptoms may be assessed, or the user may be provided with positive stimulation to alleviate the user's cognitive impairment symptoms. According to an embodiment, the first phase may be conducted in the morning, and the second phase may be conducted in the afternoon. According to another embodiment, both the first phase and the second phase may be conducted in the morning or in the afternoon. The first phase and the second phase will be described in detail with reference to FIGS. 2 to 4.

FIG. 2 is a block diagram of a management server for implementing a method according to an embodiment of the present disclosure.

Hereinafter, descriptions will be provided with reference to FIG. 1.

Referring to FIG. 2, it may be seen that the management server 200 according to an embodiment of the present disclosure includes a database 210, a communication unit 230, a processing unit 250, and an output unit 270.

The management server 200 according to an embodiment of the present disclosure may correspond to one or more processors, or may include one or more processors. Accordingly, the management server 200 and the communication unit 230, the processing unit 250, and the output unit 270 included in the management server 200 may be driven in a form included in a hardware device such as a microprocessor or a general-purpose computer system.

Each module included in the management server 200 illustrated in FIG. 2 is arbitrarily named in order to intuitively describe the representative function performed by the module, and in an actual implementation of the management server 200, each module may be named differently from as illustrated in FIG. 2.

In addition, the number of modules included in the management server 200 of FIG. 2 may vary according to an embodiment. In more detail, although FIG. 2 illustrates that the management server 200 includes a total of three modules, according to an embodiment, two or more modules may be integrated into one module, or one or more modules may be separated into two or more modules.

The database 210 stores various pieces of data necessary for the management server 200 to operate. For example, the database 210 stores an integrated management program for controlling the operation of the management server 200, and may receive and store data received by the communication unit 230 from a user terminal.

The communication unit 230 performs communication with a user terminal included in the user terminal group 110.

The processing unit 250 processes data received by the communication unit 230 and data to be transmitted. Data processed by the processing unit 250 includes data received from a user terminal or data to be transmitted to a user terminal.

In an embodiment, the processing unit 250 may combine data received by the communication unit 230 with information stored in the database 210 and process a result of the combining, or may instruct the communication unit 230 and the output unit 270 to appropriately operate to implement a method according to the present disclosure.

Functions performed by the processing unit 250 are not limited to a particular function, and although FIG.2 illustrates the processing unit 250 as a single module, the processing unit 250 may be divided into a plurality of submodules according to a process performed by the processing unit 250.

The output unit 270 receives a command from the processing unit 250 and generate and output various pieces of data.

In the present disclosure, the management server 200 may communicate with a user terminal in which the training program is installed, and control the type of information output to the user terminal. Here, an integrated management program installed and operated in the processing unit 250 of the management server 200 to control a user terminal, and a training program installed in the user terminal may function as elements for implementing a method according to the present disclosure. For example, the integrated management program operated in the management server 200 may be a program that merely stores and manages information generated from the training program operated in the user terminal, and in this case, the number of functions and size of the training program installed in the user terminal inevitably increase. On the contrary, the integrated management program operated in the management server 200 may be designed to process most of the processes according to the present disclosure, and the training program installed in the user terminal may be implemented to simply mirror the processing results from the integrated management program operated in the management server 200, and in this case, the training program installed in the user terminal may be a lightweight program capable of processing only simple interface information and login information. That is, the method according to the present disclosure may be implemented through at least one of the management server 200 and the user terminal.

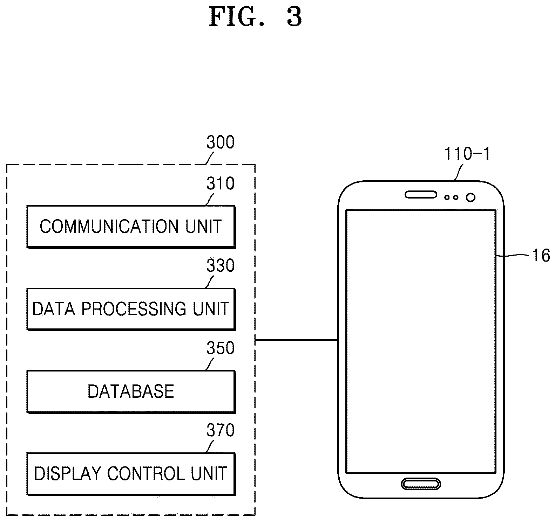

FIG. 3 is a diagram schematically illustrating a user terminal and a training program installed in the user terminal, according to an embodiment of the present disclosure.

Hereinafter, description will be provided with reference to FIG. 2, and a user terminal 110-1 of FIG. 3 is considered to be one of the terminals included in the user terminal group 110 of FIG. 2.

In FIG. 3, the user terminal 110-1 illustrated together with a training program 300 refers to an electronic device including a display 16 for displaying visual data and an interface unit (not shown) for receiving data from a user. Although FIG. 3 illustrates the user terminal 110-1 as a smart phone, the user terminal 110-1 in an implementation of the present disclosure is not limited to a smart phone, and may include a portable terminal such as a tablet PC or a netbook.

The training program 300 of FIG. 3 is not a physical device, but represents an intangible application installed in the user terminal 110-1, as a logical device. FIG. 3 illustrates that the training program 300 is included in the user terminal 110-1, however, when the training program 300 is actually implemented in the user terminal 110-1, a set of scripts composed of various instructions may be integrated into an executable program and then implemented to be installed in the user terminal 110-1. That is, the training program 300 operates to control content displayed on the display 16 of the user terminal 110-1.

FIG. 3 illustrates only components of the training program 300 that highlight features of an embodiment of the present disclosure. Thus, those of skill in the art may understand that, according to an embodiment other than the embodiment illustrated in FIG. 3, other general-purpose components may be further included in addition to the components illustrated in FIG. 3.

Referring to FIG. 3, it may be seen that the training program 300 includes a communication unit 310, a data processing unit 330, a database 350, and a display control unit 370.

The communication unit 310 may control communication with an external device. In detail, the communication unit 310 may be understood as a logical module that implements a communication function such that a training program for alleviating cognitive impairment according to the present disclosure operates normally while the user terminal 110-1 transmits and receives various pieces of data to and from the management server 200. For example, the communication unit 310 may perform a function of establishing a channel to communicate with the management server 200, and whenever there is a user request, opening a session to transmit, to the management server 200, information about the user that is input to the user terminal 110-1 and a result of executing a training algorithm according to an input by the user, or a function of receiving various pieces of data necessary for the operation of the training program 300 from the management server 200. That is, the communication unit 310 may control a physical communication module equipped in the user terminal 110-1 to operate restrictively according to a predefined command, in accordance with a process according to the present disclosure.

The data processing unit 330 may process an overall process necessary for implementing the method according to the present disclosure. The data processing unit 330 is a logical implementation (with scripts) of a processor that is a physical device, and refers to a logical module designed based on at least one of application-specific integrated circuits (ASICs), digital signal processors (DSPs), digital signal processing devices (DSPDs), programmable logic devices (PLDs), field-programmable gate arrays (FPGAs), controllers, microcontrollers, microprocessors, and other electrical units for performing a function.

For example, the data processing unit 330 may perform control, in response to a first input to the user terminal 110-1, such that a first domain training algorithm, a second domain training algorithm, and a third domain training algorithm of the first phase are output on the user terminal 110-1, determine, based on second inputs to the user terminal 110-1, whether the first domain training algorithm, the second domain training algorithm, and the third domain training algorithm of the first phase are completed, determine, based on a result of the determination, whether the first phase is normally terminated, perform control, after the first phase is terminated, in response to a third input to the user terminal 110-1, such that a first domain training algorithm, a second domain training algorithm, and a third domain training algorithm of the second phase are output on the user terminal 110-1, determine, based on fourth inputs to the user terminal 110-1, whether the first domain training algorithm, the second domain training algorithm, and the third domain training algorithm of the second phase are completed, determine, based on a result of the determination, whether the second phase is normally terminated, and perform control, according to whether the first phase and the second phase are terminated, such that a first visual indication is output on the user terminal 110-1. The above-described first phase, second phase, first region training algorithm, second region training algorithm, and third region training algorithm will be described below.

The database 350 may store various pieces of information in conjunction with hardware physically included in the user terminal 110-1, such as a memory, and in response to a request from the data processing unit 330, perform control such that information stored in the database 350 is delivered to the data processing unit 330. In addition, various pieces of data collected by the data processing unit 330 when processing data may be stored in the database 350.

When information to be output on the display 16 of the user terminal 110-1 is determined by a series of processes, the display control unit 370 performs a function of performing control such that the determined information is output. Here, the determined information is information processed by the data processing unit 330 and refers to information determined to be output by the data processing unit 330.

In more detail, the training program 300 in FIG. 3 will be described for each submodule.

First, in response to a first input to the user terminal 110-1, the data processing unit 330 may perform control such that the first region training algorithm, the second region training algorithm, and the third region training algorithm of the first phase are output on the user terminal 110-1.

Here, the first input to the user terminal 110-1 comprehensively refers to an act of initializing the training program 300 installed in the user terminal 110-1 in order for the user to alleviate cognitive impairment symptoms. For example, the user may apply a 'touch input' to a touch display provided in the user terminal 110-1 to execute the training program 300 installed in the user terminal 110-1, and here, the touch input may correspond to the first input of the present disclosure. In the present disclosure, the first input is not limited to only a tactile input to the user terminal 110-1, and thus may also include a voice input or a visual input (e.g., iris recognition) from a user.

The first phase refers to a concept for broadly dividing, into two processes, the overall process of the training program 300 for alleviating cognitive impairment symptoms provided to a user according to the present disclosure. For example, in a case in which the training program 300 needs to be divided into a first half and a second half, training algorithms performed in the first half may be included in the first phase, and training algorithms performed in the second half may be included in the second phase.

In addition, in the present disclosure, the first domain training algorithm, the second domain training algorithm, and the third domain training algorithm are considered to refer to different training algorithms. In particular, the first domain training algorithm, the second domain training algorithm, and the third domain training algorithm do not simply mean different algorithms, but mean that the methods of providing stimulation to alleviate the user's cognitive impairment symptoms may be clearly distinguished and classified into different domains.

For example, the first domain training algorithm may be an algorithm capable of quantifying and assessing the attentional control of a user receiving the training. The first domain training algorithm includes two or more detailed training algorithms for assessing or improving the attentional control of a user, and this will be described below with reference to Table 1. Hereinafter, the first domain training algorithm may be referred to as simply "attention domain training".

As another example, the second domain training algorithm may be an algorithm capable of quantifying and assessing the associative skills of a user receiving the training. The second domain training algorithm includes two or more detailed training algorithms for assessing or improving the associative skills of a user, and this will be described below with reference to Table 1. Hereinafter, the second domain training algorithm may be referred to as simply "association domain training".

As another example, the third domain training algorithm may be an algorithm capable of quantifying and assessing the integrative skills of a user receiving the training. The third domain training algorithm includes two or more detailed training algorithms for assessing or improving the integrative skills of a user, and this will be described below with reference to Table 1. Hereinafter, the third domain training algorithm may be referred to as simply "integration domain training".

**[Table 1]**

| **Training name** | **Phase** | **Functional domain** | **Subdomain** | **Strategy** | **Number of stages** | **Difficulty level adjustment method** |
|---|---|---|---|---|---|---|
| Memorizing aloud | First half | Attention | Working memory | Reading aloud and memorizing | 7 | Increasing the number of words |
| Recalling and speaking | First half | Association | Verbal imagery | Visualization | 7 | Increasing the number of words |
| Memorizing with a story | First half | Integration | Verbal integration | Memorizing by connecting stories | 7 | Increasing the number of words |
| Memorizing repeatedly | Second half | Attention | Working memory | Repetition | 7 | Increasing the number of words |
| Clapping | Second half | Attention | Speed | Increasing speed | 7 | Reducing time limit |
| Memorizing an order | Second half | Attention | Selective attention | Doing one at a time | 7 | Increasing the number of words |
| Finding words | Second half | Attention | Selective attention | Doing one at a time | 7 | Increasing the number of words |
| Memorizing appearance | Second half | Association | Verbal imagery | Visualization | 7 | Increasing the number of words |
| Throwing words | Second half | Association | Visuospatial imagery | Visualization | 7 | Increasing the number of words |
| Memorizing absurdly | Second half | Integration | Verbal integration | Memorizing by connecting stories | 7 | Increasing the number of words |
| Combining into one | Second half | Integration | Visuospatial integration | Memorizing by connecting pictures | 7 | Increasing the number of words |

Table 1 is for describing in detail attention domain training, association domain training, and integration domain training of the present disclosure. Table 1 relates to an embodiment of the present disclosure, and according to an embodiment, other training than those shown in Table 1 may be added, or some of the training shown in Table 1 may be removed. Referring to Table 1, it may be seen that there are a total of 11 training algorithms. In the present disclosure, the attention domain training includes a total of five detailed training algorithms, the association domain training includes a total of three detailed training algorithms, and the integration domain training includes a total of three detailed training algorithms.

Referring to Table 1, it may be seen that each detailed training algorithm is preset as to when it needs to be performed, either in the first half or the second half. For example, "Recalling and speaking", corresponding to the attention domain training, may be arranged to be performed only in the first half (the first phase), and is set not to be performed in the second half (the second phase). As another example, "Memorizing absurdly", corresponding to the integration domain training, may be arranged to be performed only in the second half (the second phase), and is set not to be performed in the first half (the first phase).

In the present disclosure, the data processing unit 330 may establish a training schedule for the first phase by arranging one or more training algorithms in the first phase, considering the overall information set as shown in Table 1. The user may execute the training program 300, log in by entering user information, and then perform training with a training algorithm for the first phase determined by the data processing unit 330.

Hereinafter, the 11 training algorithms described in Table 1 will be described in detail.

First, "Memorizing aloud" is training belonging to the attention domain training, and refers to training for increasing working memory capacity by prompting the user to read aloud and memorize a passage while gradually increasing information to be memorized. "Memorizing aloud" is configured to provide words to be memorized by the user one by one through the display 16 of the user terminal 110-1, and when providing the words, provide one word at a time with a voice. The user is prompted to repeat the provided word three times, and when a target stimulus is presented through the display of the user terminal 110-1, the user may perform an action according to the target stimulus. Here, the target stimulus may include conditions such as "aloud", "slowly", or "quickly".

In addition to the target stimulus, a distractor stimulus may also be provided. In the present disclosure, the target stimulus refers to a stimulus provided without adding new content in order to increase the difficulty of training, and the distractor stimulus refers to a stimulus provided in the form of adding new content in order to further increase the difficulty of training. The user receives a higher score for more actively responding to the target stimulus and for not responding to the distractor stimulus.

**[Table 2]**

| Stage | Configuration | Number of target stimuli | Total number of presented stimuli | Proportion of target stimuli |
|---|---|---|---|---|
| 1 | 2 training words | 10 | 20 | 50 % |
| 2 | 3 training words | 11 | 20 | 55 % |
| 3 | 4 training words | 12 | 20 | 60 % |
| 4 | 5 training words | 13 | 20 | 65 % |
| 5 | 6 training words | 14 | 20 | 70 % |
| 6 | 7 training words | 15 | 20 | 75 % |
| 7 | 8 training words | 16 | 20 | 80 % |

Table 2 shows a difficulty level configuration for "Memorizing aloud". "Memorizing aloud" consists of a total of 7 stages. Referring to Table 2, it may be seen that, as the difficulty level increases, the number of words for training increases, and simultaneously, the percentage of target stimuli (i.e., a score for assessment as a successful training result) also increases.

Next, "Recalling and speaking" is training belonging to the association domain training, and refers to a training algorithm to prompt the user to utilize experience and imagination to facilitate memory. "Recalling and speaking" is training that provides the user with one word, then instructs the user to think about what comes to mind after seeing the word, and then prompt the user to speak about his/her thought for a predetermined time (e.g., 20 seconds).

**[Table 3]**

| Stage | Configuration |
|---|---|
| 1 | 2 training words |
| 2 | 3 training words |
| 3 | 4 training words |
| 4 | 5 training words |
| 5 | 6 training words |
| 6 | 7 training words |
| 7 | 8 training words |

Table 3 shows a difficulty level configuration for "Recalling and speaking". "Recalling and speaking" consists of a total of 7 stages. Referring to Table 3, it may be seen that, as the difficulty level increases, the number of words for training increases.

Next, "Memorizing with a story" is training belonging to the integration domain training, and refers to training that provides the user with an example of writing a story, then provides the user with a predetermined number of words, and prompt the user to create a story of a predetermined length by using all of the provided words without omission. When there are any missing words, the user may be given one or two additional opportunities.

**[Table 4]**

| Stage | Overall configuration | Further division | |
|---|---|---|---|
| 1 | 2 training words | | |
| 2 | 3 training words | | |
| 3 | 4 training words | 2 training words | 2 training words |
| 4 | 5 training words | 2 training words | 3 training words |
| 5 | 6 training words | 3 training words | 3 training words |
| 6 | 7 training words | 3 training words | 4 training words |
| 7 | 8 training words | 4 training words | 4 training words |

Table 4 shows a difficulty level configuration for "Memorizing with a story". "Memorizing with a story" consists of a total of 7 stages. Referring to Table 4, it may be seen that, as the difficulty level increases, the number of words for training increases, and a further division of the training words is added.

"Memorizing Repeatedly" is training belonging to the attention domain training, in which when a word is presented with accompanying text and audio, the user is prompted to repeatedly speak the presented word to memorize it. After the user memorizes a predetermined number of words for each difficulty level, the user is prompted to speak all the memorized words at once, and as the number of missing words increases, the score decreases.

**[Table 5]**

| Stage | Overall configuration |
|---|---|
| 1 | 2 training words |
| 2 | 3 training words |
| 3 | 4 training words |
| 4 | 5 training words |
| 5 | 6 training words |
| 6 | 7 training words |
| 7 | 8 training words |

Table 5 shows a difficulty level configuration for "Memorizing repeatedly". "Memorizing repeatedly" consists of a total of 7 stages. Referring to Table 5, it may be seen that, as the difficulty level increases, the number of words for training gradually increases.

"Clapping" is training belonging to the attention domain training, and refers to training that induces the user's memory processing speed to be improved while gradually shortening the time taken for clapping. After the user memorizes a predetermined number of words for each difficulty level, the user is prompted to speak all the memorized words at once, and as the number of missing words increases, the score decreases. In the training, after the user checks and memorizes words provided through the user terminal 110-1, when several words are presented again after a predetermined time elapses, the user is prompted to clap only when the memorized words are displayed.

**[Table 6]**

| Stage | Speed | Proportion | Number of target stimuli | Total number of presented stimuli |
|---|---|---|---|---|
| 1 | 3.0 → 2.8 | 50 % | 11 | 22 |
| 2 | 2.9 → 2.7 | 55 % | 12 | 23 |
| 3 | 2.8 → 2.6 | 60 % | 14 | 24 |
| 4 | 2.7 → 2.5 | 65 % | 16 | 25 |
| 5 | 2.6 → 2.4 | 70 % | 18 | 26 |
| 6 | 2.5 → 2.3 | 75 % | 20 | 27 |
| 7 | 2.4 → 2.2 | 80 % | 22 | 28 |

Table 6 shows a difficulty level configuration for "Clapping". "Clapping" consists of a total of 7 stages. Referring to Table 6, it may be seen that, as the difficulty level of the "Clapping" training increases, a higher level of quick thinking and accuracy are required.

"Memorizing an order" is training belonging to the attention domain training, and is a type of training for suppressing distractions and focusing only on a target. "Memorizing an order" is training in which one store is randomly selected from among a vegetable store, a fish store, and a fruit store, then a virtual customer's voice is provided in the presence of a distractor stimulus (e.g., background music), and an assesses is performed on how accurately the user memorizes an order for vegetables, fish, or fruit included in the customer's voice.

**[Table 7]**

| Stage | Total number of orders | Volume of background sound | Number of customers | Number of orders per customer | |
|---|---|---|---|---|---|
| 1 | 2 training words | | 1 | | |
| 2 | 3 training words | | 1 | | |
| 3 | 4 training words | | 1 | | |
| 4 | 5 training words | | 2 | 2 | 3 |
| 5 | 6 training words | | 2 | 3 | 3 |
| 6 | 7 training words | | 2 | 3 | 4 |
| 7 | 8 training words | | 2 | 4 | 4 |

Table 7 shows a difficulty level configuration for "Memorizing an order". "Memorizing an order" consists of a total of 7 stages. Referring to Table 7, it may be seen that, as the difficulty level of "Memorizing an order" increases, the number of training words increases, and the number of customers and the number of orders per customer increase together.

"Finding words" is training belonging to the attention domain training, and refers to training for improving the user's selective attention by suppressing distractions and focusing only on a target. "Finding words" may primarily provide the user with a category to focus on. Here, the category for the user to focus on may be randomly selected from among four categories of animals, means of transportation, jobs, and clothes. Once the category to focus on is selected, several words are provided to the user one by one at 2-second intervals, including distractor words, and the user is prompted to speak the words appropriate for the category all at once within 5 seconds.

**[Table 8]**

| Stage | Number of training words | Total number of words | Number of turns |
|---|---|---|---|
| 1 | 1 | 5 | 3 |
| 2 | 2 | 6 | 3 |
| 3 | 2 | 7 | 3 |
| 4 | 3 | 7 | 2 |
| 5 | 3 | 8 | 2 |
| 6 | 4 | 8 | 2 |
| 7 | 4 | 9 | 2 |

Table 8 shows a difficulty level configuration for "Finding words". "Finding words" consists of a total of 7 stages. Referring to Table 8, it may be seen that, as the difficulty level of "Finding words" increases, the number of training words and the total number of words increase, and the number of turns decreases.

"Memorizing appearance" is training belonging to the association domain training, and is training to enable the user to create a clear mental image through the user's verbal description. In the "Memorizing appearance" training, the user may be provided with a description target and description elements. The description elements may be composed of a total of seven elements: shape, color, pattern, material, quantity, size, and length. In the training, after receiving a description of an object, the user may imagine the object for 5 seconds and speak about the description elements one by one.

**[Table 9]**

| Stage | Overall configuration | Number of objects | Number of descriptions per object |
|---|---|---|---|
| 1 | 2 descriptions | 1 | 2 |
| 2 | 3 descriptions | 1 | 3 |
| 3 | 4 descriptions | 1 | 4 |
| 4 | 5 descriptions | 2 | 2, 3 |
| 5 | 6 descriptions | 2 | 3, 3 |
| 6 | 7 descriptions | 2 | 3, 4 |
| 7 | 8 descriptions | 2 | 4, 4 |

Table 9 shows a difficulty level configuration for "Memorizing appearance". "Memorizing appearance" consists of a total of 7 stages. Referring to Table 9, it may be seen that, as the difficulty level of "Memorizing appearance" increases, the number of objects and the number of description elements per object increase.

"Throwing words" is training belonging to the association domain training, and is training for learning a priming process of unconsciously placing information in a familiar space and then recalling the information. In this training, words to be memorized by the user are provided in text and audio, then the user is prompted to read the word aloud at least once, then an animation of throwing the words into the sea is provided to the user, then after a predetermined time elapses, the user is prompted to speak the words thrown into the sea in order.

**[Table 10]**

| Stage | Overall configuration |
|---|---|
| 1 | 2 training words |
| 2 | 3 training words |
| 3 | 4 training words |
| 4 | 5 training words |
| 5 | 6 training words |
| 6 | 7 training words |
| 7 | 8 training words |

Table 10 shows a difficulty level configuration for "Throwing words". "Throwing words" consists of a total of 7 stages. Referring to Table 10, it may be seen that, as the difficulty level of "Word Throwing" increases, the number of training words increases.

"Memorizing absurdly" is training belonging to the integration domain training, and refers to training that promotes the user's memory through a process of memorizing by creating a story. In this training, each time a word to be memorized is provided, the user is additionally provided with an integrated sentence, and when a part of the integrated sentence is presented, the user is prompted to speak the missing word from the integrated sentence.

**[Table 11]**

| Stage | Overall configuration | Further division | |
|---|---|---|---|
| 1 | 2 training words | | |
| 2 | 3 training words | | |
| 3 | 4 training words | 2 training words | 2 training words |
| 4 | 5 training words | 2 training words | 3 training words |
| 5 | 6 training words | 3 training words | 3 training words |
| 6 | 7 training words | 3 training words | 4 training words |
| 7 | 8 training words | 4 training words | 4 training words |

Table 11 shows a difficulty level configuration for "Memorizing absurdly". "Memorizing absurdly" consists of a total of 7 stages. Referring to Table 11, it may be seen that, as the difficulty level of "Memorizing absurdly" increases, the number of training words and the number of sub-sentences of an integrated sentence increase.

"Combining into one" is training belonging to the integration domain training, and refers to training that promotes the user's memory through a process of memorizing by creating a story. In this training, each time a word to be memorized is provided, the user is additionally provided with an integrated image, and when a part of the integrated image is presented, the user is prompted to speak the word corresponding to the integrated image within 5 seconds.

**[Table 12]**

| Stage | Overall configuration | Number of index words |
|---|---|---|
| 1 | 2 training words | 2 |
| 2 | 3 training words | 3 |
| 3 | 4 training words | 4 |
| 4 | 5 training words | 5 |
| 5 | 6 training words | 6 |
| 6 | 7 training words | 7 |
| 7 | 8 training words | 8 |

Table 12 shows a difficulty level configuration for "Combining into one". "Combining into one" consists of a total of 7 stages. Referring to Table 12, it may be seen that, as the difficulty level of "Combining into one" increases, the number of training words increases.

As described above with reference to Table 1 to Table 12, in the present disclosure, the first domain training algorithm (attention domain training), the second domain training algorithm (association domain training), and the third domain training algorithm (integration domain training) may be arbitrarily selected in the first phase and output on the display 16 of the user terminal 110-1, such that the user may perform training for the first phase.

Subsequently, the data processing unit 330 may determine, based on second inputs to the user terminal 110-1, whether the first domain training algorithm, the second domain training algorithm, and the third domain training algorithm of the first phase are completed, and determine, based on a result of the determination, whether the first phase is normally terminated. Here, the term 'second input' collectively refers to a series of inputs entered into the user terminal 110-1 by the user while participating in training when training algorithms of the first phase consisting of attention domain training, association domain training, and integration domain training are output on the user terminal 110-1. That is, the term 'second input' may refer to a one-time input, but it generally refers to all inputs that need to be made to complete the attention domain training, the association domain training, and the integration domain training, and thus may refer to one or more inputs to the user terminal 110-1.

After the second inputs are entered into the user terminal 110-1, the data processing unit 330 may assess results of the training sessions performed by the user, based on the second inputs. When the training sessions in the first phase are normally terminated as the user made appropriate inputs, the data processing unit 330 stores training results of the first phase in the database 350 and, if necessary, transmits the training results to the management server 200 such that the training results may be managed as metadata for the user. For example, when at least some of the second inputs by the user were inappropriate, the data processing unit 330 may determine that the training sessions in the first phase have not been normally terminated, and in this case, the training sessions of the second phase cannot be performed.

After the first phase is terminated, the data processing unit 330 may perform control, in response to a third input to the user terminal 110-1, such that attention domain training, association domain training, and integration domain training of the second phase are output on the user terminal 110-1. Here, the third input refers to an input entered into the user terminal 110-1 by the user on the same date as a first input for executing the first phase, and when the third input is entered into the user terminal 110-1, the training of the second phase may begin. However, the training of the second phase is executable only when the training of the first phase is normally terminated, and thus, according to an embodiment, when the training of the first phase is not normally terminated, the training of the second phase may not be output on the user terminal 110-1 even through the third input is entered.

In the method according to the present disclosure, completing both the training of the first phase and the training of the second phase on the same date is considered as one complete training execution. Thus, when the training of the first phase has not been normally terminated and the third input is entered, or even when the training of the first phase has been normally terminated but the date has changed due to the passage of time, the attention domain training, the association domain training, and the integration domain training of the second phase does not begin.

After performing control such that the training of the second phase to be output on the user terminal 110-1 in a state in which the training of the first phase has been normally terminated, the data processing unit 330 may determine, based on fourth inputs to the user terminal 110-1, whether the training of the second phase is completed, and determine, based on a result of the determination, whether the second phase is normally terminated. Here, the fourth inputs collectively refer to a series of inputs entered into the user terminal 110-1 during the training process of the second phase, and may include touch inputs, voice inputs, and the like by the user.

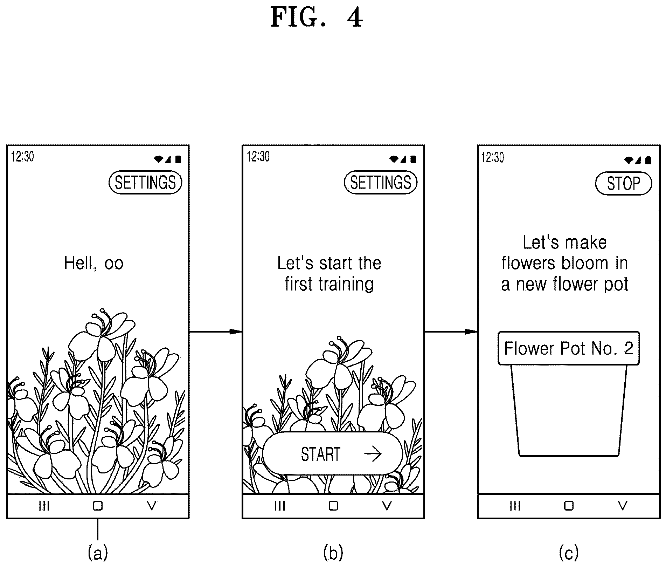

According to whether the first phase and the second phase are terminated, the data processing unit 330 may perform control such a first visual indication is output on the user terminal 110-1, through the display control unit 370. For example, based on determining that both the training of the first phase and the training of the second phase have been normally terminated, the data processing unit 330 may deliver a command to the display control unit 370 to perform control such that the first visual indication is output. As another example, when at least one of the training of the first phase and the training of the second phase has not been normally terminated, the data processing unit 330 may perform control such the first visual indication is not output. The display control unit 370 may receive a command from the data processing unit 330 and perform control such a flower of memory, which is a kind of the first visual indication, is displayed through the display of the user terminal 110-1. The flower of memory will be described below with reference to FIGS. 4 to 13.

In summary, as shown in Table 1, the data processing unit 330 arranges a total of 11 detailed training algorithms in the first phase and the second phase according to certain conditions, and performs a key function of inducing the user to perform training to alleviate cognitive impairment symptoms.

In a first embodiment, the attention domain training may be set to be arranged first in each of the first phase and the second phase. Because the attention domain training includes training to improve the user's level of attention, the data processing unit 330 may improve the user's training performance by arranging the attention domain training first among the training sessions of each of the first phase and the second phase, which are performed at intervals.

In a second embodiment, the association domain training may be a training algorithm that is set to be arranged and output immediately after the attention domain training. As a result of applying the present disclosure to several patients with cognitive impairment symptoms, it was found statistically, experimentally, and empirically that the association domain training induces the highest symptom alleviation effect in a user when provided to the user immediately after the attention domain training is finished. Thus, in the present embodiment, when generating a training curriculum of the first phase or the second phase, the data processing unit 330 may arrange the association domain training after the attention domain training.

In a third embodiment, the association domain training may be a training algorithm that is set to be arranged and output immediately before the integration domain training. As a result of applying the present disclosure to several patients with cognitive impairment symptoms, it was found statistically, experimentally, and empirically that the association domain training induces the highest symptom alleviation effect in a user when provided to the user before the integration domain training begins. Thus, in the present embodiment, when generating a training curriculum of the first phase or the second phase, the data processing unit 330 may arrange the association domain training immediately before the integration domain training.

In a fourth embodiment, the integration domain training may be a training algorithm that is set to be arranged and output immediately after the association domain training. As a result of applying the present disclosure to several patients with cognitive impairment symptoms, it was found statistically, experimentally, and empirically that the integration domain training induces the highest symptom alleviation effect in a user when provided to the user immediately after the association domain training is finished. Thus, in the present embodiment, when generating a training curriculum of the first phase or the second phase, the data processing unit 330 may arrange the integration domain training after the association domain training.

In a fifth embodiment, the association domain training may be a training algorithm that is set to be arranged immediately after the attention domain training and immediately before the integration domain training. According to the present embodiment, in order to effectively train a user with cognitive impairment symptoms, the most desirable result may be derived by configuring the attention domain training, the association domain training, and the integration domain training as one set unit, and randomly selecting only the detailed training algorithms of each domain.

In a sixth embodiment, the integration domain training may be a training algorithm that is set to be arranged immediately after the association domain training, which is performed after the attention domain training. Because the integration domain training includes detailed training algorithms that require higher-level thinking skills than the attention domain training and the association domain training, the data processing unit 330 may increase the user's training efficiency and induce a desirable training result by allowing the integration domain training to be performed only after the attention domain training and the association domain training are completed in each phase.

In a seventh embodiment, the attention domain training may be a training algorithm that is set to be output two or more times in the second phase. In the present disclosure, because the first phase and the second phase proceed on the same day, a user starting the second phase may feel a lack of physical strength or a lack of concentration, and may have a difficulty in performing the training of the second phase in good condition. According to the present embodiment, the data processing unit 330 arranges the attention domain training sessions consecutively in the second phase, and then arranges the subsequent training sessions, so as to allow the user to perform the subsequent training sessions with high concentration.

**[Table 13]**

| | **Detailed training** | **Domain** | **Note (corresponding embodiments)** |
|---|---|---|---|
| First phase | Memorizing aloud | Attention | First embodiment |
| | Recalling and speaking | Association | Second, third, and fifth embodiments |
| | Memorizing with a story | Integration | Fourth and sixth embodiments |
| Second phase | Memorizing repeatedly | Attention | First embodiment |
| | Clapping | Attention | First and seventh embodiments |
| | Throwing words | Association | Second, third, and fifth embodiments |
| | Memorizing absurdly | Integration | Fourth and sixth embodiments |

Table 13 shows an example in which the data processing unit 330 appropriately arranges detailed training algorithms to correspond to the above-described embodiments. As shown in Table 13, the numbers of training algorithms included in the first phase and the second phase may be different from each other.

As a result of applying various types of training to patients experiencing numerous cognitive impairment symptoms, the highest training effect was derived when training was divided into two phases with attention domain training, association domain training, and integration domain training efficiently arranged as in Table 13. That is, the present disclosure is an improved technology, focusing on the efficiency of arranging and providing detailed training sessions to a user based on a certain methodology, although the excellence of each of the detailed training sessions constituting the training is also important. According to the present disclosure, the training effect may be maximized by the arrangement order of training algorithms included in the first phase and the second phase.

In an alternative embodiment, the data processing unit 330 may perform control such that an assessment of the user's training result on a training algorithm is displayed in the form of a report through the user terminal 110-1 whenever a training algorithm included in the first phase and the second phase is terminated. According to the present embodiment, the user may quickly check a quantified training result in a report whenever training of each domain is completed, and thus be encouraged before performing the training of the next domain.

In addition, the data processing unit 330 may perform control such that assessments of other users' training results for a training algorithm, in addition to an assessment of the user's training result, are displayed in the form of a report through the user terminal 110-1 whenever a training algorithm included in the first phase and the second phase is terminated. According to the present embodiment, the user may check his/her training result for each domain before all training sessions of the first phase and the second phase are terminated, and participate in training more ambitiously while comparing his/her training result with other users' training results.

FIG. 4 is a diagram for describing a first visual indication that is output through a user terminal.

Hereinafter, descriptions will be provided with reference to FIG. 3.

It has already been described with reference to FIG. 3 that the first visual indication controlled by the display control unit 370 to be output on the user terminal 110-1 may be a flower of memory. The flower of memory refers to an image that embodies a flower pot, a stem grown from the flower pot, and a flower that blooms from the stem.

(a) of FIG. 4 exemplarily illustrates a screen output on the user terminal 110-1 when the user successfully logs in to a training program. In (a) of FIG. 4, the user's name and a simple greeting are displayed together with flowers of memory.

(b) of FIG. 4 is a screen that requests the user's input such that the user may start the training of the first phase. The user may apply an input to a start button such that (c) of FIG. 4 is output.

(c) of FIG. 4 schematically shows a flower pot in which a flower of memory will grow. The indication "Flower Pot No. 2" means that the user has already completed and archived "Flower Pot No. 1" by using a training program. While using the training program according to the present disclosure for 12 weeks, the user may create and archive up to 12 complete flower pots. 7 stems may grow in one flower pot, and 12 completed flower pots include a total of 84 stems. By archiving and visually checking a flower pot containing a flower of memory, the user may intuitively feel a sense of accomplishment that the user has steadily performed training for alleviating cognitive impairment symptoms. An empty flower pot is illustrated in (c) of FIG. 4, but in a case in which there is a previous training history, one or more stems may be shown together with a flower of memory.

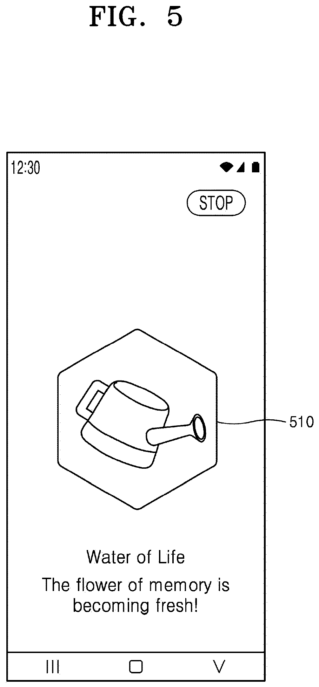

FIG. 5 is a diagram schematically illustrating an example of a badge that is obtainable by a user.

In more detail, when the user logs in to the training program through the user terminal 110-1, the display control unit 370 may detect a login history before any one of the first phase and the second phase is executed, and provide a reward based on the detected login history. In the present disclosure, the reward may be provided to the user in the form of a badge or a flower of memory.

In FIG. 5, a water badge 510 refers to a badge with a watering can engraved on a hexagonal base, and the water badge 510 may be a reward provided when the user's cumulative login count reaches a predetermined number. Hereinafter, for convenience of description, a reward provided when the cumulative login count reaches the predetermined number will be referred to as a first reward. Because the first reward is visually displayed on the user terminal 110-1, the first reward may also be referred to as a second visual indication, following the flower of memory, which is an example of the first visual indication.

The text of the water badge 510 in FIG. 5 means that "water of life", which may make the flower of memory fresh, is provided as a reward when the user's cumulative login count has reached the predetermined number, and the display control unit 370 may perform control such that the visual effect of the first visual indication, which is the flower of memory, is changed based on history information about the second visual indication. For example, when the water of life is applied, the number of petals may be greater than usual when changing from a flower bud of memory to a flower. Through the visual change of the flower of memory, the user may feel the reward of training and obtain a sense of accomplishment.

In an alternative embodiment, the predetermined number for providing the first reward to the user may correspond to a certain number of logins spaced at regular time intervals. For example, the display control unit 370 may detect a login on a fifth day, a login on a tenth day, and a login on a fifteenth day, and then perform control such that the first reward is provided.

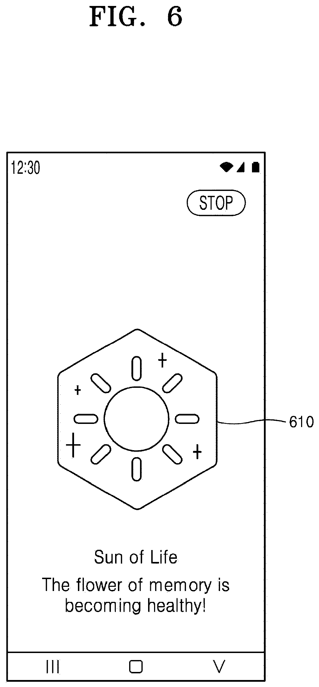

FIG. 6 is a diagram schematically illustrating another example of a badge that is obtainable by a user.

In FIG. 6, a sun badge 610 refers to a badge with a sun engraved on a hexagonal base, and the sun of the sun badge 610 is a result of embodying sunlight and sunshine. The sun badge 610 may be a reward provided when the user's consecutive login count reaches a predetermined number. Hereinafter, for convenience of description, a reward provided when the consecutive login count reaches the predetermined number will be referred to as a second reward. Because the second reward is also visually displayed on the user terminal 110-1 like the first reward, the second reward may also be referred to as a third visual indication, following the flower of memory, which is an example of the first visual indication, and the water badge, which is an example of the second visual indication.

The text of the sun badge 610 in FIG. 6 means that "sun of life", which may make the flower of memory healthy, is provided as a reward when the user's consecutive login count has reached the predetermined number, and the display control unit 370 may perform control such that the visual effect of the first visual indication, which is the flower of memory, is changed based on history information about the third visual indication. For example, when the sun of life is applied, the color of the petals of the flower of memory may be changed to a more vivid and intense color, and the user may feel the reward of training and obtain a sense of accomplishment through the visual change of the flower of memory.

In an alternative embodiment, the predetermined number for providing the first reward to the user may correspond to dates determined according to a certain rule. For example, the display control unit 370 may detect logins on 3 consecutive days, logins on 3 consecutive days, logins on 10 consecutive days, and logins on 20 consecutive days, and then perform control such that the second reward is provided.

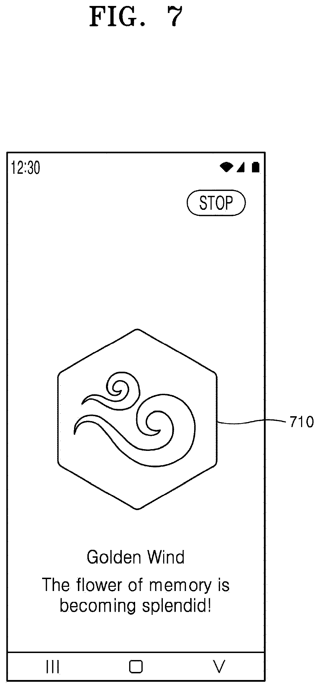

FIG. 7 is a diagram schematically illustrating another example of a badge that is obtainable by a user.

In FIG. 7, a golden wind badge 710 refers to a badge with a golden wind engraved on a hexagonal base, and the golden wind of the golden wind badge 710 is a result of embodying wind. The golden wind badge 710 may be a reward provided only when an average correct answer rate, which is an assessment result, is greater than or equal to 50 % after the training of the first phase and the second phase is normally terminated. Hereinafter, for convenience of description, a reward provided when the average correct answer rate is greater than or equal to 50 % will be referred to as a third reward. Because the third reward is also visually displayed on the user terminal 110-1 like the first reward, the third reward may also be referred to as a fourth visual indication, following the flower of memory, which is an example of the first visual indication, the water badge, which is an example of the second visual indication, and the sun badge 610, which is an example of the third visual indication.

The text of the golden wind badge 710 in FIG. 7 means that "golden wind", which may make the flower of memory splendid, is provided as a reward when the user's average correct answer rate is greater than 50 %, and the display control unit 370 may perform control such that the visual effect of the first visual indication, which is the flower of memory, is changed based on history information about the fourth visual indication. For example, when the golden wind is applied, the color of the petals of the flower of memory is changed to gold, and the user may feel the reward of training and obtain a sense of accomplishment through the visual change of the flower of memory.

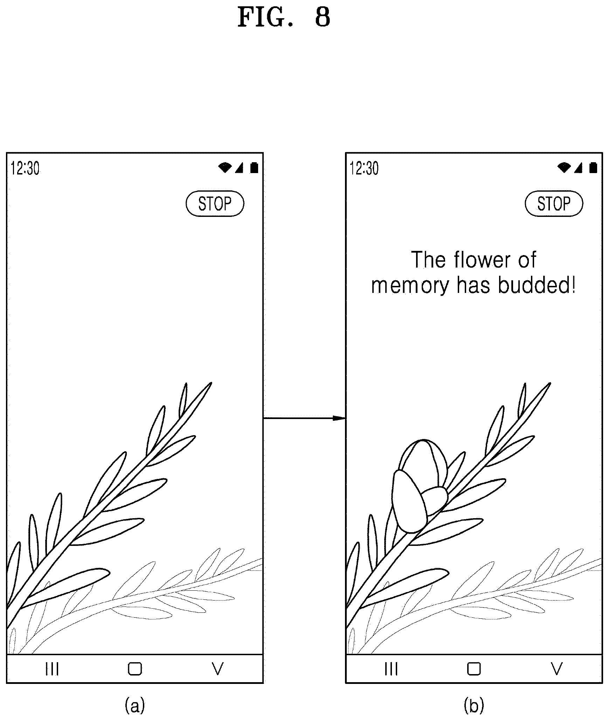

FIG. 8 is a diagram for describing a bud of a flower of memory, which is an example of the first visual indication.

The flower of memory is an example of the first visual indication, and when the user logs in and receives a flower pot, and then the first phase is normally terminated, a bud of the flower of memory is formed on the stem, and when the second phase is also normally terminated, the bud is unfurled into full bloom. FIG. 8 schematically illustrates a process, performed by the display control unit 370, of forming a bud of a flower of memory when the first phase is normally terminated.

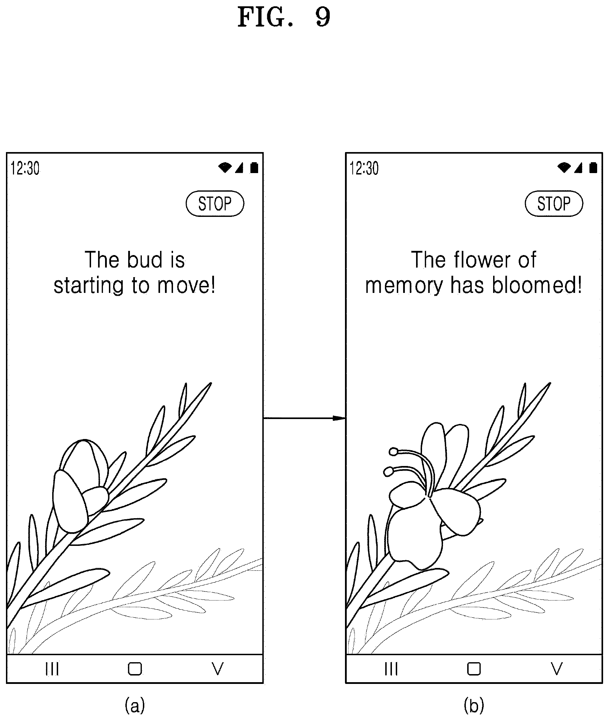

FIG. 9 is a diagram for describing a flower of memory in full bloom, which is another example of the first visual indication.

As described above, when the user has normally performed training according to the first phase, thus a bud of the flower of memory has been formed, and then the second phase is normally terminated, the flower bud as illustrated in (a) of FIG. 9 is changed to be in full bloom as illustrated in (b) of FIG. 9. Through the user terminal 110-1, the user may sequentially view the state in which there is only a flower pot, the state in which a bud of a flower of memory is formed on a stem grown from the flower pot, and the state in which the budded flower of memory has turned into full bloom, and thus have hope that cognitive impairment symptoms will be alleviated, and may actively participate in training afterward as the user feels a high sense of accomplishment and emotion.

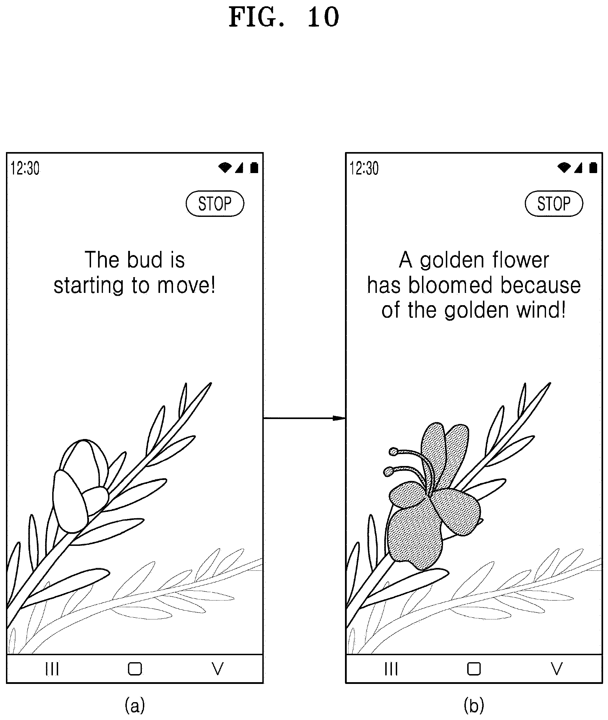

FIG. 10 is a diagram for describing a golden flower of memory in full bloom, which is another example of the first visual indication.

As described above, when the user has normally performed the first phase and the second phase, and it is determined that the average correct answer rate, which is an assessment of a training result, is greater than 50 %, the flower bud as illustrated in (a) of FIG. 10 is changed to a golden flower of memory in full bloom as illustrated in (b) of FIG. 10. Through the user terminal 110-1, the user may obtain a strong motivation for high achievement in training, while viewing the golden flower of memory in full bloom. The display control unit 370 may upgrade the budded flower of memory to a gold version and thus make it bloom, based on history information indicating that the golden wind badge 710 corresponding to the third reward, has been output as the fourth visual indication, and to provide the user with a more dramatic effect, the display control unit 370 may add a high-quality animation effect when the flower is blooming.

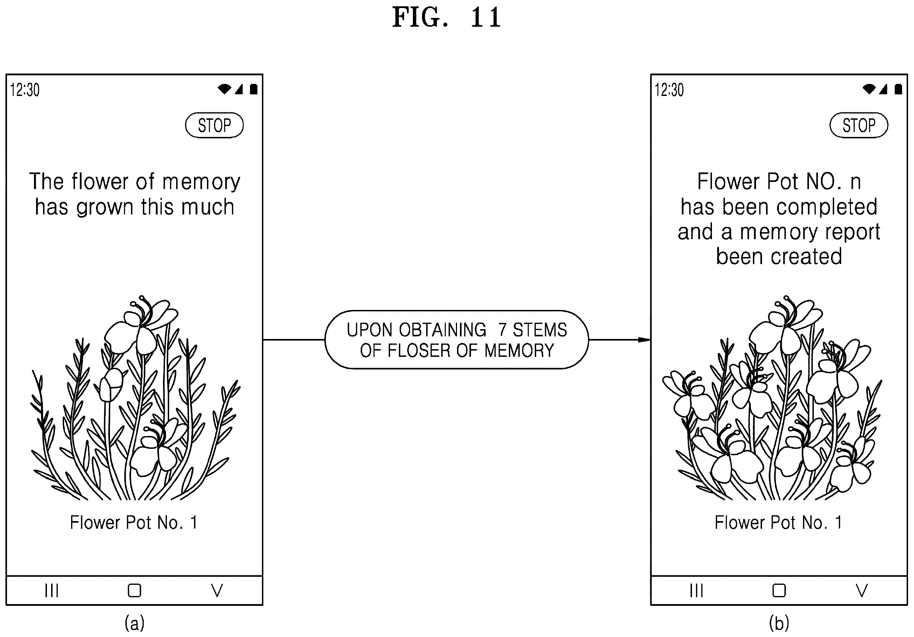

FIG. 11 is a diagram for describing archiving of a flower of memory.

(a) of FIG. 11 shows a state in which two flowers of memory are in full bloom, and one flower of memory is budded. That is, it may be interpreted that the user has normally completed the training according to the first phase and the second phase for two days, and performed only the training according to the first phase for one day.

In addition, (b) of FIG. 11 shows a state in which seven flowers of memory are in full bloom from seven stems, and it may be interpreted that the user has normally completed the training according to the first phase and the second phase for a week without taking a day off.

A stem grows from a flower pot whenever a new day starts, and if training is not performed on that day, the flower of memory is never formed on the stem, and thus, stems may no longer grow from the flower pot in both (a) and (b) of FIG. 11, and this flower pot, named Flower Pot No. 1, is archived and thus remains permanently. The user may experience aesthetic beauty and obtain a sense of accomplishment, while viewing the archived flower pot. When Flower Pot No. 1 is archived, Flower Pot No. 2 is newly provided on the next day, and a new flower of memory may be formed in Flower Pot No. 2 based on a degree of training participation of the user.

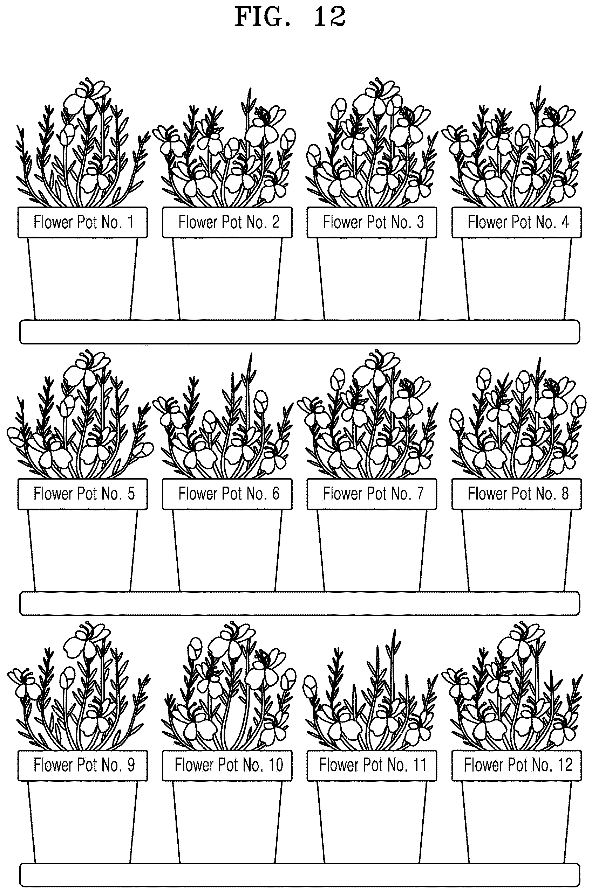

FIG. 12 is a diagram for describing another embodiment of archiving of a flower of memory described above with reference to FIG. 11.

While using the training program according to the present disclosure for 12 weeks, the user may create and archive up to 12 complete flower pots. 7 stems may grow in one flower pot, and 12 completed flower pots include a total of 84 stems. By archiving and visually checking a flower pot containing a flower of memory, the user may intuitively feel a sense of accomplishment that the user has steadily performed training for alleviating cognitive impairment symptoms.

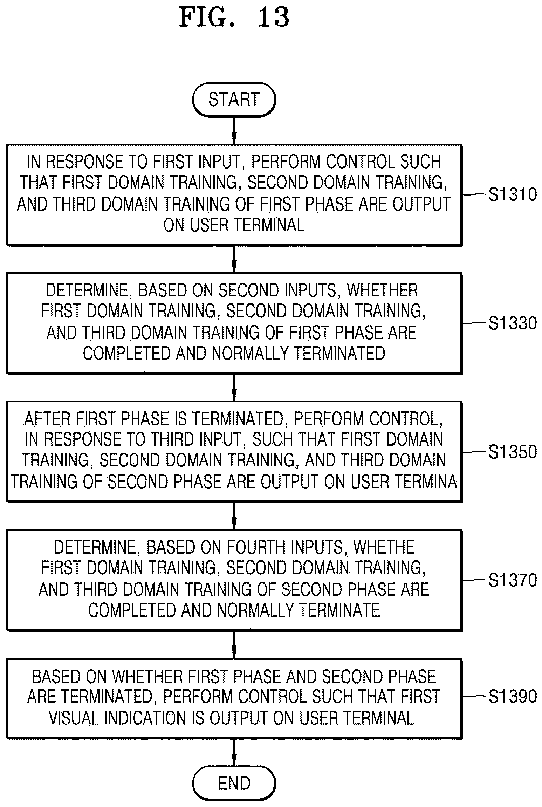

FIG. 13 is a flowchart of an example of a method according to the present disclosure.

The method according to FIG. 13 may be implemented by at least one of the management server 200 or the training program 300 described above with reference to FIGS. 1 to 12. Hereinafter, for convenience of description, the subject for implementing each method will be referred to as the training program 300.

The training program 300 may perform control, in response to a first input to a user terminal, such that a first domain training algorithm, a second domain training algorithm, and a third domain training algorithm of a first phase are output on the user terminal (S1310).

The training program 300 may determine, based on second inputs to the user terminal, whether the first domain training algorithm, the second domain training algorithm, and the third domain training algorithm of the first phase are completed, and determine, based on a result of the determination, whether the first phase is normally terminated (S1330).

After the first phase is terminated, the training program 300 may perform control, in response to a third input to the user terminal, such that a first domain training algorithm, a second domain training algorithm, and a third domain training algorithm of a second phase are output on the user terminal (S1350).

The training program 300 may determine, based on fourth inputs to the user terminal, whether the first domain training algorithm, the second domain training algorithm, and the third domain training algorithm of the second phase are completed, and determine, based on a result of the determination, whether the second phase is normally terminated (S1370).

The training program 300 may perform control, based on whether the first phase and the second phase are terminated, such that a first visual indication is output on the user terminal (S1390).

According to the present disclosure, it is possible to provide a training algorithm for dramatically improving cognitive impairment symptoms of a patient with a cognitive impairment, in various forms.

According to the present disclosure, it is possible to significantly reduce social costs required to manage patients with dementia.

According to the present disclosure, unlike related-art technology, a memory formation process of a patient with cognitive impairment may be stimulated in units of memory through imagery, semantic, and integration training, and working memory skills and processing speed may be comprehensively improved.

A training program constructed according to the present disclosure may effectively stimulate a brain domain in charge of a user's cognitive function, resulting in improvement of the thickness of the cerebral cortex and cognitive function. In the brain of a user who experienced the training program according to the present disclosure, an increase in brain volume, including changes in the entire white matter, was confirmed through a DT1 image.

According to the present disclosure, symptoms of a patient with mild cognitive impairment may be significantly alleviated.

According to the present disclosure, it is possible to prevent or diagnose in advance dementia, the most feared disease of the elderly.

According to the present disclosure, it is possible to significantly reduce social costs required to manage patients with dementia.

According to the present disclosure, unlike related-art technology, a memory formation process of a patient with cognitive impairment may be stimulated in units of memory through imagery, semantic, and integration training, and working memory skills and processing speed may be comprehensively improved.

The embodiments of the present disclosure described above may be implemented as a computer program that may be executed through various components on a computer, and such a computer program may be recorded in a computer-readable medium. In this case, the medium may include a magnetic medium, such as a hard disk, a floppy disk, or a magnetic tape, an optical recording medium, such as a compact disc read-only memory (CD-ROM) or a digital video disc (DVD), a magneto-optical medium, such as a floptical disk, and a hardware device specially configured to store and execute program instructions, such as read-only memory (ROM), random-access memory (RAM), or flash memory.

In addition, the computer program may be specially designed and configured for the present disclosure or may be well-known to and usable by those skilled in the art of computer software. Examples of the computer program may include not only machine code, such as code made by a compiler, but also high-level language code that is executable by a computer by using an interpreter or the like.

Particular executions described herein are merely examples and do not limit the scope of the present disclosure in any way. For the sake of brevity, related-art electronics, control systems, software and other functional aspects of the systems may not be described in detail. Furthermore, line connections or connection members between elements depicted in the drawings represent functional connections and/or physical or circuit connections by way of example, and in actual applications, they may be replaced or embodied with various suitable additional functional connections, physical connections, or circuit connections. In addition, no item or component is essential to the practice of the present disclosure unless the item or component is specifically described as being "essential" or "critical".

The term 'the' and other demonstratives similar thereto in the specification of the present disclosure (especially in the following claims) should be understood to include a singular form and plural forms. Furthermore, recitation of ranges of values herein are merely intended to serve as a shorthand method of referring individually to each separate value falling within the range, unless otherwise indicated herein, and each separate value is incorporated into the specification as if it were individually recited herein. Finally, the operations of the methods described herein may be performed in any suitable order unless otherwise indicated herein or otherwise clearly contradicted by context. The present disclosure is not limited to the described order of the operations. The use of any and all examples, or exemplary language (e.g., 'and the like") provided herein, is intended merely to better illuminate the present disclosure and does not pose a limitation on the scope of the present disclosure unless otherwise claimed. Also, numerous modifications and adaptations will be readily apparent to those skilled in the art without departing from the spirit and scope of the present disclosure.

## Claims

1. A method of alleviating cognitive impairment based on a user terminal, the method comprising:
performing control, in response to a first input to a user terminal, such that a first domain training algorithm, a second domain training algorithm, and a third domain training algorithm of a first phase are output on the user terminal;
determining, based on second inputs to the user terminal, whether the first domain training algorithm, the second domain training algorithm, and the third domain training algorithm of the first phase are completed, and determining, based on a result of the determining, whether the first phase is normally terminated;
after the first phase is terminated, performing control, in response to a third input to the user terminal, such that a first domain training algorithm, a second domain training algorithm, and a third domain training algorithm of a second phase are output on the user terminal;
determining, based on fourth inputs to the user terminal, whether the first domain training algorithm, the second domain training algorithm, and the third domain training algorithm of the second phase are completed, and determining, based on a result of the determining, whether the second phase is normally terminated; and
performing control, based on whether the first phase and the second phase are terminated, such that a first visual indication is output on the user terminal.

2. The method of claim 1, wherein the first domain training algorithm, the second domain training algorithm, and the third domain training algorithm each comprise two or more detailed training algorithms.

3. The method of claim 1, wherein the first domain training algorithm is an algorithm set to be arranged first in each of the first phase and the second phase.

4. The method of claim 1, wherein the first domain training algorithm, the second domain training algorithm, and the third domain training algorithm are set to be sequentially output on the user terminal, from the first domain training algorithm to the third domain training algorithm.

5. The method of claim 1, wherein the first input for executing the first phase and the third input for executing the second phase are inputs entered into the user terminal on a same date.

6. The method of claim 1, wherein the first domain training algorithm of the second phase is output two or more times within the second phase.

7. The method of claim 1, wherein the first domain training algorithm of the second phase is set to comprise two or more different detailed training algorithms to be consecutively output on the user terminal.

8. The method of claim 1, wherein, each time a training algorithm included in the first phase and the second phase is terminated, an assessment of a training result of a user for the training algorithm is displayed through the user terminal.

9. The method of claim 8, wherein, each time the training algorithm included in the first phase and the second phase is terminated, assessments of training results of other users for the training algorithm, in addition to the assessment of the training result of the user, are displayed through the user terminal.

10. The method of claim 1, wherein an arrangement order of training algorithms included in the first phase and the second phase is preset to maximize a training effect.

11. The method of claim 1, wherein the performing of the control such that the first visual indication is output comprises, based on the first phase and the second phase having been normally terminated on a same date consecutively for one week, integrating and outputting seven instances of the first visual indication, which are output for respective seven dates of the one week.

12. The method of claim 1, wherein the first visual indication is changed in response to an assessment of a training result in the first phase and the second phase.

13. The method of claim 1, wherein the first phase is performed in a morning, and
the second phase is performed in an afternoon.

14. A computer-readable recording medium having stored therein a program for executing the method of claim 1.

15. A device for alleviating cognitive impairment based on a user terminal, the device comprising:
a memory storing at least one program; and
a processor configured to execute the at least one program to perform an operation,
wherein the processor is further configured to perform control, in response to a first input to a user terminal, such that a first domain training algorithm, a second domain training algorithm, and a third domain training algorithm of a first phase are output on the user terminal, determine, based on second inputs to the user terminal, whether the first domain training algorithm, the second domain training algorithm, and the third domain training algorithm of the first phase are completed, determine, based on a result of the determining, whether the first phase is normally terminated, perform control, after the first phase is terminated and in response to a third input to the user terminal, such that a first domain training algorithm, a second domain training algorithm, and a third domain training algorithm of a second phase are output on the user terminal, determine, based on fourth inputs to the user terminal, whether the first domain training algorithm, the second domain training algorithm, and the third domain training algorithm of the second phase are completed, determine, based on a result of the determining, whether the second phase is normally terminated, and perform control, based on whether the first phase and the second phase are terminated, such that a first visual indication is output on the user terminal.
